Europäisches Patentamt

European Patent Office

Office européen des brevets

(19)

(11) Veröffentlichungsnummer: **0 000 001**
**B1**

(12) **EUROPÄISCHE PATENTSCHRIFT**

(21) Anmeldenummer: **78200013.7**

(22) Anmeldetag: **01.06.78**

(51) Int. Cl.³: **F 28 D 15/00 F 24 J 3/04**

(54) **Thermische Wärmepumpe**

(30) Priorität: **02.09.77 DE 2739689**

(43) Veröffentlichungstag der Anmeldung:
**20.12.78 Patentblatt 78/01**

(45) Bekanntmachung des Hinweises auf die Patenterteilung:
**07.01.81 Patentblatt 81/01**

(84) Benannte Vertragsstaaten:
**BE CH FR GB NL SE**

(56) Entgegenhaltungen:
**DE - A - 2 161 506**
**FR - A - 2 025 459**
**US - A - 3 532 159**
**US - A - 3 568 762**
**US - A - 3 913 665**
**US - A - 3 965 970**
**US - A - 4 018 269**

(73) Patentinhaber: **EUROPÄISCHE ATOMGEMEINSCHAFT (EURATOM)**
**Bâtiment Jean Monnet Plateau du Kirchberg Boîte Postale 1907**
**Luxembourg (LU)**

(72) Erfinder: **Busse, Claus Adolf, Dr.**
**22, Via Piaggio**
**I - 21038 Arolo di Leggiuno (Varese) (IT)**

(74) Vertreter: **Wey, Hans-Heinrich, Dipl.-Ing., et al**
**Patentanwälte Müller-Börner Wey & Körner**
**Widenmayerstrasse 49**
**D - 8000 München 22 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

## Thermische Wärmepumpe

Um Wärme von einem tieferen auf ein höheres Temperaturniveau zu heben, muß ein Kompensationsprozeß Anwendung finden, so daß die Gesamtentropie aller beteiligten Stoffe nicht abnimmt. Bei den üblichen Wärmepumpen wird ein Kompensationsprozeß mit Arbeitsverbrauch angewandt; der Arbeitsverbrauch beruht auf dem Betrieb eines Kompressors.

Der Publikation von Nesselmann, "Zur Theorie der Wärmetransformation", (Wiss. Veröffentlichungen des Siemens-Konzerns, *12* (1933), S. 89—109) ist entnehmbar, den Kompensationsprozeß auch durch Wärme anzutreiben, und zwar derart, daß bei einer mittleren Temperatur $T_0$ Wärme eingespeist wird und diese sowohl bei einer tieferen Temperatur $T_1$ als auch bei einer höheren Temperatur $T_2$ wieder abgegeben wird.

Eine thermische Wärmepumpe, welche mit fossilen Brennstoffen oder Abfallwärme höherer Temperatur betrieben wird und als Verdichteraggregat eine Heißflüssigkeits-Strahlpumpe aufweist, ist bereits aus der Veröffentlichung von Ch. Mostofizadeh in "Elektrowärme international" Edition A 35 (1977) A 1, S. A35—A36, bekanntgeworden. In dieser Heißflüssigkeits-Strahlpumpe wird der auf der Saugseite befindliche gesättigte Dampf mit Hilfe der unter hohem Druck stehenden heißen Flüssigkeit verdichtet und auf eine höhere Temperatur gebracht. Dabei sollen die von Dampfstrahlpumpen bekannten hohen Stoßverluste dadurch vermieden werden, daß der Saug- und der Treibstrom erst auf annähernd gleiche Geschwindigkeit und Temperatur expandiert und dann vermischt werden. Abgesehen von den praktischen Regelproblemen, die die Einhaltung dieser Bedingung zwangsläufig mit sich bringen, ist die Anordnung wegen der zusätzlichen Reibungsverluste durch den relativ hohen Anteil von Flüssigkeit im Dampf äußerst problematisch. Derartige Wärmepumpen haben daher wesentliche Nachteile insofern, als das Arbeitsmedium auf eine relativ hohe Temperatur erhitzt und ein Teil desselben mittels einer Druckpumpe auf den notwendig hohen Betriebsdruck gebracht werden muß und infolge erheblicher Reibungsverluste der Wirkungsgrad relativ niedrig ist.

Der Erfindung liegt die Aufgabe zugrunde, unter Anwendung der vorgenannten Theorie eine einfach gestaltete Wärmepumpe zu schaffen, die durch Wärme antreibbar ist. Eine derartige thermische Wärmepumpe wird somit durch das temperaturgefälle zwischen $T_0$ und $T_1$ angetrieben, wobei der thermische Wirkungsgrad sich aus folgender Beziehung errechnen läßt:

$$\eta \equiv \frac{\dot{Q}_2}{\dot{Q}_0} \leq \frac{1 - \dfrac{T_1}{T_0}}{1 - \dfrac{T_1}{T_2}}.$$

wobei $\dot{Q}_0$ der aufgenommene Wärmestrom und $\dot{Q}_2$ der Nutzwärmestrom bei der Temperatur $T_2$ ist.

Derart arbeitende thermische Wärmepumpen sind von großem Interesse zur Ausnutzung der durch Sonneneinstrahlung hervorgerufenen Temperaturdifferenzen, insbesondere zum Zwecke des Aufheizens von Wasser oder anderen Medien für Heizungszwecke, zur Bereitstellung von Warmwasser u.dgl.

Die erfindungsgemäß ausgebildete thermische Wärmepumpe besteht aus einem Wärmerohr, in welchem der zwischen der Wärmeübertragungszone zur Wärmezufuhr und der Wärmeübertragungszone zur Wärmeabfuhr befindliche Dampfkanal einen sich über seine Länge ändernden, die Strömungsgeschwindigkeit des Dampfes zunächst erhöhenden und dann erniedrigenden Querschnitt aufweist und sich im Bereich der erhöhten Dampfgeschwindigkeit eine weitere, dritte Wärmeübertragungszone mit Wärmezufuhr oder -abfuhr befindet. Durch die letztgenannten Maßnahmen wird ein an sich bekanntes Wärmerohr zu einer Wärmepumpe der in Betracht kommenden Art.

Aus der US-PS 35 32 159 ist ein Wärmerohr bekanntgeworden, welches einen starkwandigen zentralen zylindrischen Einsatz aufweist, zwischen welchem und der Wandung des Wärmerohres ein Ringspalt mit über die ganze Länge konstantem Querschnitt für die Rückführung des Arbeitsmediums vorhanden ist und in dessen Mitte ein Strömungskanal angeordnet ist, in welchem an der Stelle des Übergangs von einem engeren Querschnitt zu einem weiteren eine Düse angeordnet ist, um den Wirkungsgrad des Wärmerohres zu verändern, jedoch kann dieses vorbekannte Wärmerohr nicht als Wärmepumpe eingesetzt werden.

Bei einer Ausführungsform des Gegenstands der Erfindung besteht die thermische Wärmepumpe aus einem Wärmerohr, in dressen Dampfkanal zwischen den Wärmeübertragungszonen für die Wärmezu- bzw. -abfuhr an den beiden Enden des Wärmerohres ein die Dampfgeschwindigkeit ändernder Verdrängungskörper angeordnet ist.

Der vor dem Verdrängungskörper befindliche Bereich ist der Verdampferbereich "V", in welchem bei einer mittleren Temperatur $T_0$ der Wärmestrom $\dot{Q}_0$ zugeführt wird. Der Bereich etwa in der Mitte des Verdrängungskörpers ist der sogenannte Treibkondensatorbereich "TK", in welchem bei einer mittleren Temperatur $T_1$, die unterhalb der Temperatur $T_0$ liegt, ein Teil des Dampfes kondensiert, wobei der Wärmestrom $\dot{Q}_1$ abgeführt wird. Hinter dem Verdrängungskörper befindet sich der Nutzkondensatorbereich "NK", in welchem bei einer mittleren Temperatur $T_2$ der Restdampf kondensiert und der Nutzwärmestrom $\dot{Q}_2$ abgegeben wird. Das im Treibkondensatorbereich "TK" und im Nutzkondensatorbereich "NK" anfallende Kondensat wird durch eine geeignete, an sich bekannte Kapillarstruktur, mit welcher die Innenwand des Wärmerohres in üblicher Weise ausgekleidet ist, zum Verdampferbereich "V" zurückgeführt.

Auch kann die Betriebsweise der vorbeschriebenen Ausführungsform der Wärmepumpe gemäß der Erfindung derart abgeändert werden, daß sie thermodynamisch umgekehrt wird, was dazu führt, daß der Treibkondensatorbereich dann zu einem zweiten Verdampferbereich wird. Dadurch kann eine relativ große Wärmemenge von einer tiefen Temperatur zu einer mittleren Temperatur transportiert werden.

Ausführungsbeispiele der erfindungsgemäßen thermischen Wärmepumpe gehen aus der nachfolgenden Beschreibung und zweckmäßige Ausgestaltungen der Erfindung aus den Unteransprüchen hervor.

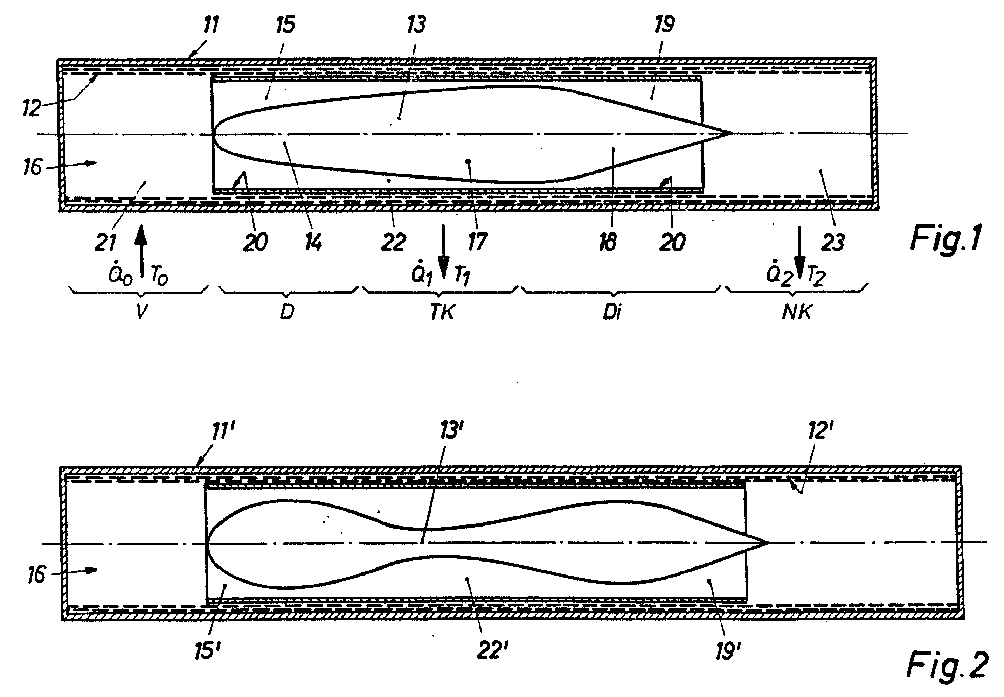

Die beiden Figuren 1 und 2 der Zeichnungen zeigen in schematischer Darstellung eine thermische oder Dampfstrahl-Wärmepumpe mit Unterschallströmung im einen Falle und mit Überschallströmung im zweiten Falle.

Die Wärmepumpe für Unterschallströmung nach Fig. 1 besteht aus einem Wärmerohr 11, an dessen Innenwand eine Kapillarstruktur 12 angeordnet ist. Im Innenraum des Wärmerohres 11 befindet sich in gleichmäßigem Abstand von der Kapillarstruktur 12 der Verdrängungskörper 13, der in seinem vorderen Bereich 14 eine Form besitzt, so daß zwischen ihm und der Kapillarstruktur 12 eine Düse 15 gebildet ist, in welcher sich der Querschnitt des Dampfkanals 16 verkleinert. Hinter der Düse verringert sich der Querschnitt des Dampfkanals 16 zwischen dem Mittelstück 17 des Verdrängungskörpers 13 und der Kapillarstruktur 12 infolge der Form des Verdrängungskörpers geringfügig. In diesem Bereich wird durch Kühlung ein Teil des Dampfes kondensiert. Beim gebräuchlichen Wärmerohr stellt sich dabei ein Druck- und Temperaturanstieg ein, der mit der Theorie der Wärmerohre in übereinstimmung steht (vgl. z.B. "Heat pipes" von Dunn und Reay, Abschnitt 2.5.5, Fig. 2.16 und 2.17). Dieser Druck- und Temperaturanstieg wird vorliegend durch die Querschnittsabnahme des Dampfkanals im wesentlichen unterbunden. Der hintere Teil 18 des Verdrängungskörpers 13 ist kegelförmig ausgebildet, so daß sich der Querschnitt des Dampfkanals 16 dem Öffnungswinkel des Kegels entsprechend erweitert und einen Diffusor 19 bildet.

Vorteilhafterweise ist die Kapillarstruktur 12 im Bereich des Verdrängungskörpers 13 im Hinblick auf die hohen Druckunterschiede längs der Wärmepumpe mit einer dünnwandigen Abdeckung 20 versehen, die zur Vermeidung eines Abhebens infolge Unterdrucks ausreichend fest mit der Kapillarstruktur bzw. dem Rohrkörper des Wärmerohres 11 verbunden sein muß. Sie hat den Zweck, das anfallende Kondensat vermittels der Scherwirkung des Dampfstromes auf der Abdeckung zunächst in die Nutzkondensatorzone zu treiben, in welcher der Druck höher ist als in der Verdampferzone, wo er seinerseits höher ist als in der Treibkondensatorzone.

Die thermische Wärmepumpe mit Überschallströmung nach Fig. 2 hat grundsätzlich den gleichen Aufbau wie diejenige nach Fig. 1. Der wesentliche Unterschied besteht darin, daß der Verdrängungskörper 13' derart geformt ist, daß die Düse 15' wie auch der Diffusor 19' einen konvergenten und einen divergenten Teil aufweisen, wobei der Übergang von der Unterschall- zur Überschallströmung jeweils im Bereich der engsten Stelle erfolgt.

Die Wirkungsweise der erfindungsgemäßen thermischen Wärmepumpe ist folgende:

Im Verdampfer 21 wird bei einer mittleren Temperatur $T_0$ der Wärmestrom $\dot{Q}_0$ zugeführt. In der durch den vorderen Teil des Verdrängungskörpers 13,13' gebildeten Düse 15,15' wird der Dampf expandiert. Hierbei kühlt er sich ab und kondensiert teilweise im anschließenden Treibkondensator 22,22' bei einer mittleren Temperatur $T_1 < T_0$, wobei der Wärmestrom $\dot{Q}_1$ abgeführt wird. Die kinetische Energie des kondensierten Dampfes verbleibt im Restdampf, so daß dessen spezifische kinetische Energie ansteigt. Die üblicherweise in einem Kondensator bei Unterschall- wie auch bei Überschallströmung auftretenden Temperaturänderungen können durch die Expansion des Dampfes im Treibkondensator 22,22' vermieden werden, und zwar durch entsprechende Verkleinerung des Dampfkanalquerschnitts. Im anschließenden Diffusor 19,19' wird der Dampf komprimiert, wobei sich dessen kinetische Energie in Druck umwandelt und die Temperatur des Dampfes ansteigt. Im sich an den Diffusor 19,19' anschließenden Nutzkondensator 23 wird bei einer mittleren Temperatur $T_2$ der Restdampf kondensiert, wobei der Nutzwärmestrom $\dot{Q}_2$ abgegeben wird. Da die spezifische kinetische Energie des Dampfes beim Eintritt in den Diffusor 19,19' höher liegt als beim Austritt aus der Düse 15,15' können im Nutzkondensator 23 höhere Temperaturen als im Verdampfer 21 erzielt werden.

Das im Treibkondensator 22,22' und im Nutzkondensator 23 anfallende Kondensat wird über die Kapillarstruktur 12,12' an der Innenwand des Wärmerohres 11,11' zum Verdampfer 21 zurückgeführt.

Für eine thermische Wärmepumpe mit einer Wärmezufuhr von $\dot{Q}_0 = 1$ kw ist folgende Dimensionierung vorzusehen:

| | |
|---|---|
| Wärmeträger: | $H_2O$ |
| Wandmaterial: | Cu |
| $T_0$: | 30°C |
| $T_1$: | 25°C |
| $T_2$: | 35°C |
| Dampfkanal-Querschnitt im Verdampfer: | 6,7 cm² |
| Dampfkanal-Querschnitt am Ausgang Düse: | 1,9 cm² |
| Dampfkanal-Querschnitt am Ausgang Treibkondensator: | 1,0 cm² |
| Machzahl am Ausgang der Düse: | 0,69 |
| Machzahl am Ausgang des Treibkondensators: | 0,97 |

Die mit Unterschallströmung erreichbaren Temperaturdifferenzen betragen nur wenige Prozent der Absoluttemperatur, wie sich aus den vorstehenden Daten ergibt. Mit der thermischen Wärmepumpe mit Überschallströmung lassen sich größere Temperaturdifferenzen erzielen.

Es ist auch eine Ausführungsform der thermischen Wärmepumpe möglich, bei welcher eine Unterschalldüse nach Fig. 1 verwendet wird, der Übergang zur Überschallströmung im Treibkondensator erfolgt und anschließend ein Überschalldiffusor nach Fig. 2 benutzt wird.

Das Kondensat wird in bekannter Weise mittels der Kapillarstruktur an der Innenwand des Wärmerohres zum Verdampfer zurückgeführt. Die Rückführung erfolgt im wesentlichen aufgrund der Kapillarkräfte, die gegebenenfalls durch die Schwerkraft unterstützt werden können. Es ist aber auch möglich, wie erwähnt, zum Antrieb der Flüssigkeitsströmung in der Kapillarstruktur zusätzlich den höheren Druck im Nutzkondensator auszunutzen.

Weiterhin ist es möglich, den abgedeckten Teil der Kapillarstruktur im Inneren des Wärmerohres durch ein oder mehrere Röhrchen, Kanäle od.dgl. zu ersetzen, die gegebenenfalls auch auf der Außenseite des Wärmerohres angeordnet sein können, um die Dampfströmung möglichst wenig zu beeinträchtigen.

Zweckmäßigerweise wird der Verdrängungskörper im Innenraum des Wärmerohres auf einem axial angeordneten Tragstab gelagert, der vorzugsweise thermisch isoliert ausgebildet ist oder aus Wärme schlecht leitendem Material besteht.

Weiterhin kann es vorteilhaft sein, in der Nutzkondensatorzone und gegebenenfalls auch in der Verdampferzone etwa kegelförmige Verdrängungskörper anzuordnen, deren Grundflächen den Stirnflächen des Wärmerohres zugekehrt sind.

Vorteilhafterweise verwendet man ein zylindrisches Wärmerohr mit Kapillarstruktur-Auskleidung und Abdeckung, in welchem ein Verdrängungskörper mit der gewünschten Querschnittsform angeordnet ist. Man kann selbstverständlich auch einen Verdrängungskörper verwenden, der zumindest in seinem mittleren Teil zylindrisch ausgebildet ist, so daß dann die Querschnitte des Dampfkanals in den verschiedenen Bereichen durch die Wände des Wärmerohres bestimmt sind. Diese Lösung ist jedoch weniger vorteilhaft. Die beschriebenen und dargestellten Ausführungsformen haben wesentliche Vorteile, da Verluste durch Grenzschichtablösung im Treibkondensator und Diffusor und Temperaturverluste durch eine größere Wärmeübertragungsfläche im Treibkondensator vermieden werden; überdies sind die konstruktive Form und die Herstellbarkeit einfach sowie stabil.

Bei einem praktischen Anwendungsbeispiel zum Zwecke optimaler Nutzbarmachung von Solarenergie könnte das Wärmerohr derart angeordnet sein, daß die der thermischen Wärmepumpe im Verdampferbereich zugeführte Wärmemenge $\dot{Q}_0$ von Sonnenstrahlen stammt; die Wärmemenge $\dot{Q}_1$ wird durch ein Kühlmittel agbeführt, beispielsweise in einem den Sonnenstrahlen nicht ausgesetzten Bereich, und die im Nutzkondensator anfallende Wärmemenge $\dot{Q}_2$ könnte zur Aufheizung eines Nutzmediums benutzt werden.

Die erfindungsgemäße thermische Wärmepumpe bietet den Vorteil geringer Verluste, einer kleinen und einfachen Konstriktion sowie der Wartungsfreiheit, woraus sich geringe Anschaffungs- und Betriebskosten ergeben.

Schließlich kann die veränderliche Ausgestaltung des Querschnitts des Dampfkanals des Wärmerohrs auch dadurch erzielt werden, daß der Verdrängungskörper fortgelassen und statt dessen das Wärmerohr querschnittsmäßig entsprechend der erforderlichen Kanalkonfiguration veränderlich ausgebildet wird und somit die gleichen Strömungseffekt erzielt werden wie im Falle der beschriebenen Ausführungsbeispiele.

**Patentansprüche**

1. Thermische Wärmepumpe, gekennzeichnet durch ein Wärmerohr (11), in welchem der zwischen der Wärmeübertragungszone zur Wärmezufuhr und der Wärmeübertragungszone zur Wärmeabfuhr befindliche Dampfkanal (16) einen sich über seine Länge ändernden, die Strömungsgeschwindigkeit des Dampfes zunächst erhöhenden und dann erniedrigenden Querschnitt aufweist und daß sich im Bereich der erhöhten Dampfgeschwindigkeit eine weitere, dritte Wärmeübertragungszone mit Wärmezufuhr oder -abfuhr befindet.

2. Thermische Wärmepumpe nach Anspruch 1, dadurch gekennzeichnet, daß im Dampfkanal (16) zwischen den Wärmeübertragungszonen für die Wärmezu- bzw. -abfuhr an den beiden Enden des Wärmerohrs (11) ein die Dampfgeschwindigkeit ändernder Verdrängungskörper (13) angeordnet ist.

3. Thermische Wärmepumpe nach Anspruch 2, dadurch gekennzeichnet, daß sich bei Wärmeabfuhr in der dritten Wärmeübertragungszone die Querschnittsfläche des Dampfkanals (16) längs des vorderen Teils (14) des Verdrängungskörpers (13) in Strömungsrichtung düsenartig verkleinert, längs seines mittleren Teils (17) geringfügig verkleinert und längs seines hinteren Teils (18) diffusorartig erweitert, wobei die Machzahl der Dampfströmung an jeder Stelle des Dampfkanals (16) kleiner als 1,0 ist.

4. Thermische Wärmepumpe nach Anspruch 2, dadurch gekennzeichnet, daß sich bei Wärmezufuhr in der dritten Wärmeübertragungszone die Querschnittsfläche des Dampfkanals (16) längs des vorderen Teils· des Verdrängungskörpers (13) in Strömungsrichtung düsenartig verkleinert, längs seines mittleren Teils (17) geringfügig vergrößert und längs seines hinteren Teils (18) diffusorartig erweitert, wobei die Machzahl der Dampfströmung an jeder Stelle des Dampfkanals (16) kleiner als 1,0 ist.

5. Thermische Wärmepumpe nach Anspruch 1 oder 2, dadurch gekennzeichnet, daß die Düse (15) und der Diffusor (19) jeweils aus einem konvergenten und einem divergenten Teil bestehen, wobei die Machzahl der Dampfströmung im Bereich der höchsten Dampfgeschwindigkeit über 1,0 liegt.

6. Thermische Wärmepumpe nach Anspruch 5, dadurch gekennzeichnet, daß die Düse eine Unterschalldüse (15') und der Diffusor ein Überschalldiffusor (19') ist, wobei der Übergang von der Unterschall- zur Überschallströmung im Bereich der erhöhten Dampfgeschwindigkeit (TK) erfolgt.

7. Thermische Wärmepumpe nach einem oder mehreren der Ansprüche 1 bis 6, dadurch gekennzeichnet, daß die Kapillarstruktur (12) längs der Innenwand des Wärmerohres (11) etwa über die Länge des Verdrängungskörpers (13) gegen den Dampfkanal (16) mit einer Abdekkung (20) versehen ist.

8. Thermische Wärmepumpe nach einem oder mehreren der Ansprüche 1 bis 7, dadurch gekennzeichnet, daß im Bereich des Verdrängungskörpers (13) die Kapillarstruktur (12) ganz oder teilweise durch innerhalb oder außerhalb der Rohrwandung des Wärmerohrs (11) angeordnete Röhrchen, Kanäle od.dgl. ersetzt ist.

9. Thermische Wärmepumpe nach einem oder mehreren der Ansprüche 1 bis 8, dadurch gekennzeichnet, daß der Verdrängungskörper (13) auf einem thermisch isolierten axialen Haltestab gelagert ist.

10. Thermische Wärmepumpe nach einem oder mehreren der Ansprüche 1 bis 9, dadurch gekennzeichnet, daß am hinteren und bzw. oder vorderen Ende des Wärmerohres ein etwa kegelförmiger Verdrängungskörper angeordnet ist, dessen Grundfläche jeweils der Stirnwand des Wärmerohres (11) zugekehrt ist.

**Revendications**

1. Pompe à chaleur thermique, caractérisée par le fait qu'elle comprend un tube thermique (11) dans lequel le conduit de vapeur (16) compris entre la zone de transmission de chaleur pour l'apport de chaleur et la zone de transmission de chaleur pour la cession de chaleur présente une section transversale variant le long de ce conduit, destinée en premier lieu à accélérer la vitesse d'écoulement de la vapeur et, en second lieu, à freiner cette dernière, et dans lequel se trouve en outre une zone de transmission de chaleur supplémentaire, avec apport ou cession de chaleur, dans le secteur de vitesse d'écoulement accélérée de la vapeur.

2. Pompe à chaleur thermique suivant la revendication 1, caractérisée par le fait qu'un éjecteur (13) modifiant la vitesse d'écoulement de la vapeur est disposé dans le conduit de vapeur (16) du tube thermique entre les zones de transmission de chaleur pour l'apport ou la cession de chaleur aux deux extrémités du tube thermique (11).

3. Pompe à chaleur thermique suivant la revendication 2, caractérisée par le fait que, lors de la cession de chaleur dans la troisième zone de transmission de chaleur, la section transversale du conduit de vapeur (16) le long de la partie amont (14) de l'éjecteur (13) diminue en forme de tuyère dans le sens de l'écoulement, diminue de façon restreinte le long de la partie médiane (17) de l'éjecteur (13) et s'élargit en forme de diffuseur à la partie postérieure (18) de l'éjecteur (13), le nombre de Mach indiquant la vitesse de l'écoulement de vapeur se situant au-dessous de 1,0 pour chaque position du conduit de vapeur (16).

4. Pompe à chaleur thermique suivant la revendication 2, caractérisée par le fait que, lors de l'apport de chaleur dans la troisième zone de transmission de chaleur, la section transversale du conduit de vapeur (15) diminue en forme de tuyère le long de la partie amont de l'éjecteur (13) dans le sens de l'écoulement, diminue de façon restreinte le long de la partie médiane (17) de l'éjecteur et s'élargit en forme de diffuseur à la partie aval du diffuseur (18), le nombre de Mach indiquant la vitesse de l'écoulement se situant au-dessous de 1,0 pour chaque position de conduit de vapeur (16).

5. Pompe à chaleur thermique suivant la revendication 1 ou 2, caractérisée par le fait que la tuyère (15) et le diffuseur (19) sont constitués respectivement par une partie convergente et par une partie divergente, le nombre de Mach indiquant la vitesse de la vapeur pénétrant dans la zone d'écoulement de la vapeur à vitesse maximale (TK) se situant au-dessus de 1,0.

6. Pompe à chaleur thermique suivant la revendication 5, caractérisée par le fait que la tuyère (15) est une tuyère subsonique et que le diffuseur est en diffuseur supersonique (19'), le passage de la vitesse d'écoulement s'effectuant du domaine subsonique au domaine supersonique dans la zone d'écoulement accéléré de la vapeur (TK).

7. Pompe à chaleur thermique suivant une ou plusieurs des revendications 1 à 6, caractérisée par le fait que la structure capillaire (12) disposée sur la paroi intérieure du tube thermique (11) est revêtue d'une chemise (20) sur une longueur correspondant approximativement à celle de l'éjecteur (13), parallèlement au conduit de vapeur (16).

8. Pompe à chaleur thermique suivant une ou plusieurs des revendications 1 à 7, caractérisé par le fait que la structure capillaire (12) est partiellement ou entièrement remplacée dans la zone approximative de l'éjecteur (13) par des tubulures, conduits ou autres canalisations similaires disposés à l'intérieur ou à l'extérieur de la paroi tubulaire du tube thermique (11).

9. Pompe à chaleur thermique suivant une ou plusieurs des revendications 1 à 8, caractérisée par le fait que l'éjecteur (13) est fixé sur une potence axiale isolée thermiquement.

10. Pompe à chaleur thermique suivant une ou plusieurs des revendications 1 à 9, caractérisée par le fait qu'à l'extrémité postérieure et/ou antérieure du tube thermique (11) est disposé un éjecteur de forme sensiblement cônique dont la surface de base est tournée vers la surface frontale du tube thermique (11).

## Claims

1. Thermal heat pump, characterized by a heat pipe (11) in which the vapor passage (16) located between the heat transfer zone to the heat supply and the heat transfer zone to the heat removal has a cross section which varies across its length and which increases the velocity of the vapor flow to begin with and then decreases it and that a further, third heat transfer zone with heat supply or heat removal is located in the area of the increased vapor velocity.

2. Thermal heat pump according to claim 1, characterized in that a displacement body (13) altering the vapor velocity is arranged in the vapor passage (16) between the heat transfer zones for heat supply and removal at the two ends of the heat pipe (11).

3. Thermal heat pump according to claim 2, characterized in that during heat removal the cross sectional surface of the vapor passage (16) in the third heat transfer zone is reduced in a jet-like manner in the direction of flow along the front portion (14) of the displacement body (13), that it is slightly reduced along the middle portion (17) of the displacement body and that it is widened in a diffuserlike manner along the rear portion (18) of the displacement body, wherein the Mach number of the vapor flow lies below 1,0 at each point of the vapor passage (16).

4. Thermal heat pump according to claim 2, characterized in that during heat supply the cross sectional surface of the vapor passage (16) in the third heat transfer zone is reduced in a jet-like manner in the direction of flow along the front portion of the displacement body (13), that it is slightly enlarged along the middle portion (17) of the displacement body and that it is widened in a diffuserlike manner along the rear portion (18) of the displacement body (13), wherein the Mach number of the vapor flow lies below 1,0 at each point of the vapor passage.

5. Thermal heat pump according to claim 1 or 2, characterized in that the jet (15) and the diffuser (19) each consist of a convergent and a divergent part, wherein the Mach number of the vapor flow in the area of the highest vapor velocity (TK) lies above 1,0.

6. Thermal heat pump according to claim 5, characterized in that the jet is a subsonic jet (15) and the diffuser (19') is a supersonic diffuser, wherein the transition from the subsonic to the supersonic flow occurs in the area of the increased vapor velocity (TK).

7. Thermal heat pump according to one or more of claims 1 to 6, characterized in that the capillary structure (12) along the inner wall of the heat pipe (11) is provided with a cover (20) against the vapor passage (16) roughly over the length of the displacement body (13).

8. Themal heat pump according to one or more of claims 1 to 7, characterized in that the capillary structure (12) is totally or partially replaced in the area of the displacement body (13) by tubes, ducts and the like, which are arranged inside or outside of the pipe wall of the heat pipe (11).

9. Thermal heat pump according to one or more of claims 1 to 8, characterized in that the displacement body (13) is placed on a thermally insulated axial support member.

10. Thermal heat pump according to one or more of claims 1 to 9, characterized in that an approximately cone-shaped displacement body is arranged at the rear and/or front end of the heat pipe, the base surface of which displacement body faces the front wall of the heat pipe (11) in each case.

Fig.1

Fig.2

0 000 001